# EUROPEAN PATENT SPECIFICATION

(11) **EP 3 019 466 B1**
(45) Date of publication and mention of the grant of the patent: **06.09.2017**
(21) Application number: 14736743.7
(22) Date of filing: 03.07.2014
(51) Int. Cl.: C07C 205/53

(54) **PROCESS FOR PREPARING 8-ARYLOCTANOIC ACIDS**
VERFAHREN ZUR HERSTELLUNG VON 8-ARYL-OCTANONSÄUREN
PROCÉDÉ DE PRÉPARATION D'ACIDES 8-ARYLOCTANOÏQUES

(30) Priority: 11.07.2013 IT MI20131170
(43) Date of publication of application: 18.05.2016
(73) Proprietor: F.I.S.- Fabbrica Italiana Sintetici S.p.A., 36075 Montecchio Maggiore (VI) (IT)
(72) Inventor: MARAGNI, Paolo, I-46030 Virgilio (MN) (IT); BENAGLIA, Maurizio, I-20100 Milano (IT); COTARCA, Livius, I-33052 Cervignano del Friuli (UD) (IT); VERZINI, Massimo, I-37042 Caldiero (VR) (IT)
(74) Representative: Leganza, Alessandro
(86) International application number: PCT/EP2014/064158
(87) International publication number: WO 2015/003988

(56) References cited:
- WO-A1-2011/070459

## Description

The present invention relates to a process for preparing aliskiren and, more particularly, to an improved method for synthesizing a β-amino alcohol or a corresponding lactone derivative thereof, these intermediates being used in the preparation of aliskiren.

8-Aryloctanoic acids having 2S,4S,5S,7S configuration of general formula 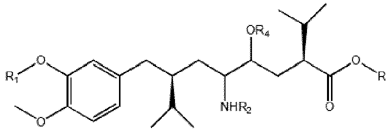 are known as excellent antihypertensive agents which interfere with the renin-angiotensin system.

Aliskiren hemifumarate is a direct inhibitor of renin and is indicated in the treatment of arterial hypertension optionally in combination with amlodipine, hydrochlorothiazide or both.

Aliskiren hemifumarate, described chemically as (2S,4S,5S,7S)-N-(2-carbamoyl-2-methylpropyl)-5-amino-4-hydroxy-2,7-diisopropyl-8-[4-methoxy-3-(3-methoxypropoxy)-phenyl]-octanamide hemifumarate, of formula 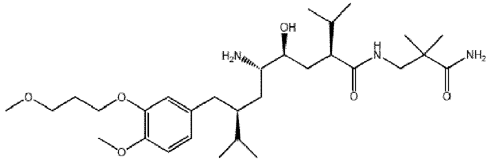 is a white or slightly yellow crystalline powder with a molecular weight of 609.8, which is soluble in phosphate buffer, n-octanol and highly soluble in water.

The literature discloses various processes for preparing aliskiren and/or derivatives thereof. Patent EP 0678503 (Ciba-Geigy Corporation) describes δ-amino-γ-hydroxy-ω-aryl-alkanoic acids and/or derivatives thereof and salts thereof with renin-inhibiting properties that are useful as antihypertensive medicaments.

The preparation of δ-amino-γ-hydroxy-ω-arylalkanoic acid amides comprises the reaction of a compound of formula II with an amine of formula III with formation of the corresponding amide bond and removal of the protecting group, if present; in particular, preferred compounds of formula II are the lactone compounds of formula IIc 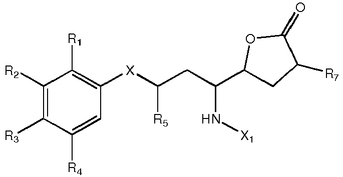 the preparation of which involves, inter alia, reacting a compound of formula XXV 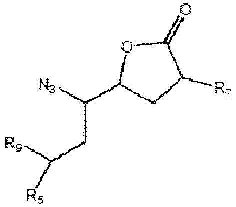 in which R₉ is a formyl group, with a compound of formula XIV 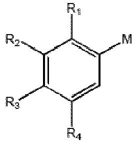 in which M is a metal radical (for example Mg-halogen) to give a compound of formula XVI optionally protected on the benzyl hydroxyl group; reduction of the azide to amine and optional introduction of the protecting group X₁.

Similarly, patent EP 1303478 (Speedel Pharma AG) describes a method for preparing aliskiren in which the double bond of the key amide intermediate of 2,7-dialkyl-8-aryl-4-octenoic acid is, simultaneously, halogenated in position 5 and hydroxylated in position 4 to give a lactone ring which is then opened by amidation and whose halogen group is replaced with an azide subsequently converted to an amine.

Tetrahedron Letters 46 (2005), 6337-6340 describes a convergent synthesis of aliskiren via the use of the compound [(1S,3S)-1-((2S,4S)-4-isopropyl-5-oxo-2-tetrahydrofuryl)-3-[4-methoxy-3-(3-methoxypropoxy-)benzyl]-4-methylpentyl]-tert-butyl carbamate (indicated as Segment AB, Boc-lactone hereinbelow) as key intermediate; the preparation of said intermediate takes place via a coupling reaction of the Grignard reagent derived from Segment B with Segment A as in the scheme reported hereinbelow 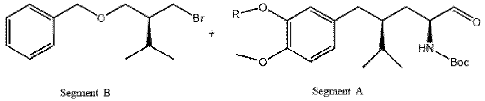 followed by oxidative lactonization. The opening reaction of the lactone ring with an aminoamide synthon (Segment C) gives the desired pharmaceutical active principle.

International patent application WO 2003/103653 (Elan Pharmaceuticals Inc.) describes aliskiren and derivatives thereof in the treatment of Alzheimer's disease. The application involves the preparation of lactone intermediates that are useful in the preparation of the therapeutically active agents (see in particular the compounds of formula IId on page 117 and the various preparative examples).

International patent application WO 2006/024501 (Novartis Pharma GMBH) describes alternative syntheses of aliskiren and intermediates thereof according to the scheme reported hereinbelow

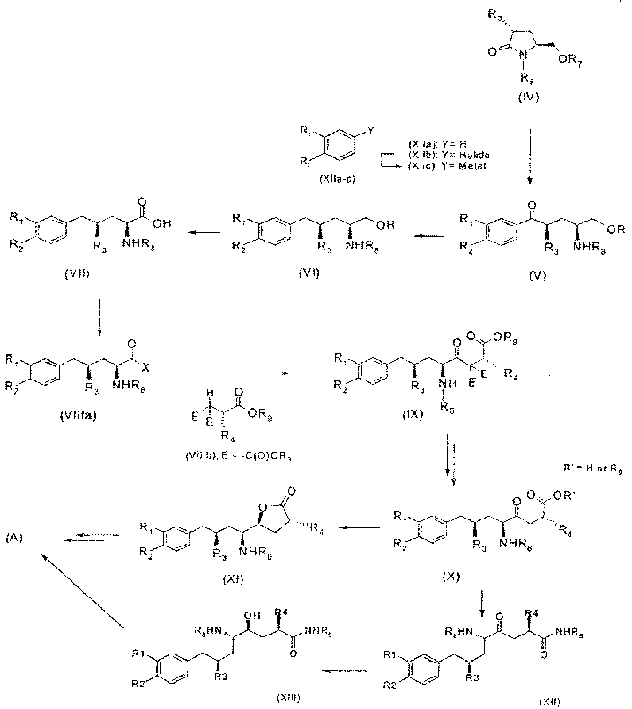

In particular, the synthetic examples 10 and 11 describe the preparation of the lactone intermediate [(1S,3S)-1-((2S,4S)-4-isopropyl-5-oxo-2-tetrahydrofuryl)-3-[4-methoxy-3-(3-methoxypropxy)-benzyl]-4-methylpentyl]-tert-butyl carbamate and the related coupling with the compound 3-amino-2,2-propionamide to give aliskiren.

The synthetic approach to aliskiren and/or intermediates thereof via C(5)-nitro derivatives is reported in some international patent applications.

International patent application WO 2011/127797 (Unitris Biopharma Co.) describes the preparation of aliskiren and intermediates thereof; in particular, a description is given of the nitro-aldol reaction of the intermediates of formulae II and III to give the nitro compound I according to the following scheme 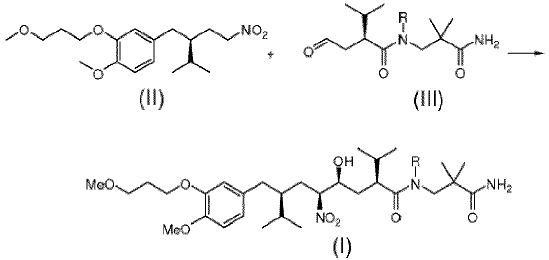 which is converted into aliskiren by reduction.

However, said patent application describes the nitroaldol reaction between the nitroalkane of formula II with an aldehyde synthon of formula III which contains the amide function already installed and which therefore requires a further step for protection (and subsequent deprotection) of the amide nitrogen (R = p-methoxybenzyl). It should then be observed that the description of the nitro-aldol of formula (I) reaction product is rather insufficient since it reports only a weight yield on the crude product and the analytical data for the molecular ion obtained by MS analysis, but without any data regarding the chemical purity and above all regarding the stereoisomeric purity of the isolated product.

A further element that is considered as being missing from the description of the nitro-aldol reaction step according to the teaching of WO 2011/127797 is the described use of a limited excess (1.07 eq.) of nitroalkane of formula II relative to the aldehyde substrate of formula III. This runs contrary both to that reported in the literature and that which we observed during the study of the reaction.

Furthermore, the synthesis of the nitroalkane of formula II performed according to the teachings of WO 2011/127797 is problematic, in particular, in the step of alkylation between the iodoalkyl derivative and the nucleophilic substrate (anion) generated by reacting nitromethane with a base.

International patent application WO 2011/070459 (Carbodesign LLC.) describes novel processes and novel intermediates that are useful in the synthesis of pharmaceutical active ingredients, in particular, renin inhibitors such as aliskiren. The invention describes, inter alia, optically active 8-aryloctanoic derivatives; in particular, aliskiren is prepared via chiral nitro derivatives according to the scheme reported hereinbelow

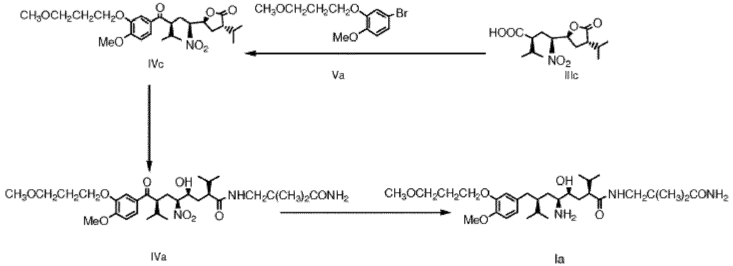

International patent application WO 2009/080773 (DSM IP Assets B.V.) describes a convergent synthesis of renin inhibitors and of intermediates that are useful in the preparation thereof. Said patent application describes the reaction of an aryl compound of formula 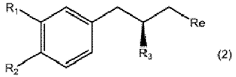 with a derivative of formula 7 or 8 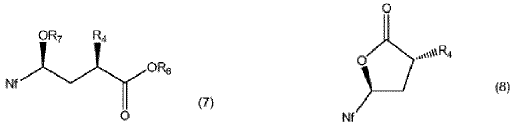 in which Nf is, inter alia, a nitro group; to give the intermediate of formula 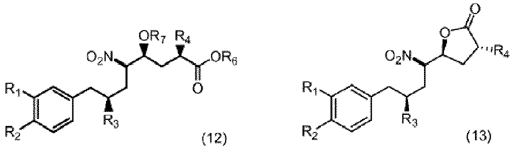 which is appropriately treated so as to obtain the desired diastereoisomeric purity at the chiral carbon in position 5, reduced to the optionally protected amine to ensure its inertness with respect to the amidation reaction able to give the finished product or a protected analog thereof.

It is known in the art that the synthesis of molecular structures such as 8-aryloctanoic acids having the configuration 2S,4S,5S,7S of general formula defined above is challenging to a person skilled in the art on account of the four asymmetric carbon atoms which give rise to a mixture of 16 stereoisomers (in the case of asymmetric substitutions) or to a mixture of 10 stereoisomers (in the case of symmetric substitutions).

The synthetic approaches described are identifiable as multi-step syntheses in which the overall yield and also the yields of the individual steps are highly penalizing from the industrial viewpoint.

In addition, producing the correct steric configuration of the chiral centers, in particular, of those in positions C(4) and C(5) into which are introduced the hydroxyl and amine groups, respectively, proves to be a critical step of the synthetic process.

Therefore, being known in the art the essential role of the compound [(1S,3S)-1--((2S,4S)-4-isopropyl-5-oxo-2-tetrahydrofuryl)-3-[4-methoxy-3-(3-methoxy-propoxy)-benzyl]-4-methylpentyl]-tert-butyl carbamate as a key intermediate in the synthesis of 8-aryloctanoic acids of configuration 2S,4S,5S,7S endowed with renin-inhibiting action, it would be desirable to study alternative methods which allow said intermediate to be prepared in good yields and under conditions that are more favorable from the point of view of the industrial application of the process.

We have now surprisingly found an improved process for the synthesis of [(1S,3S)-1-((2S,4S)-4-isopropyl-5-oxo-2-tetrahydrofuryl)-3-[4-methoxy-3-(3-methoxypropoxy)-benzyl]-4-methylpentyl]-tert-butyl carbamate, a key intermediate in the preparation of aliskiren, via the corresponding nitro derivative, which makes it possible to overcome the drawbacks presented by the processes described in the prior art.

Therefore, it is an object of the present invention a process for preparing a compound of formula 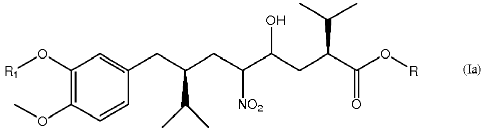 wherein R is hydrogen, benzyl and an optionally branched (C₁-C₆)-alkyl group and R₁ is a (C₁-C₄)-alkoxy-(C₁-C₄)-alkyl group; or a lactone derivative thereof of formula 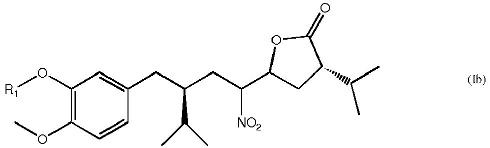 wherein R₁ is defined above, which comprises
a. reacting a compound of formula 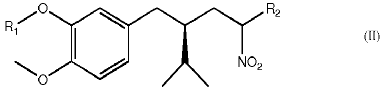 wherein R₁ is defined above and R₂ is hydrogen, carboxy, cyano and alkoxy-carbonyl; with a compound of formula 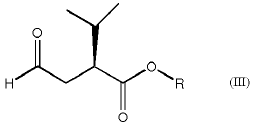 wherein R is defined above; in the presence of a basic species or a catalyst,
b. optional lactonizing a compound of formula Ia to a compound of formula Ib.

The compounds of formulae II and III may be prepared according to common synthetic techniques from commercially available substrates.

The compound of formula II in which R₂ is hydrogen, for example, may be prepared according to the procedure reported in international patent application WO 2011/127797, cited above, which involves two steps according to the scheme reported hereinbelow:

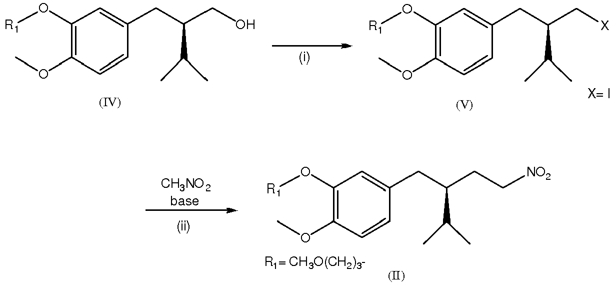

Step (i): conversion of the corresponding hydroxyalkyl derivative of formula IV into the corresponding halo derivative of formula V (the iodo derivative is described, X=I).

Step (ii): reaction of the halo derivative V with nitromethane in the presence of a base to give the compound of formula II (in which R₂ is hydrogen).

However, the alkylation reaction of Step (ii) between a haloalkyl derivative and the nucleophilic substrate (anion) generated from nitromethane, when reproduced by us, proved to be problematic and did not give satisfactory yields.

Alternatively, the inventors performed an efficient synthesis of the compound of formula II (in which R₂ is hydrogen) which involves a sequence of steps all in high yield according to the scheme reported hereinbelow:

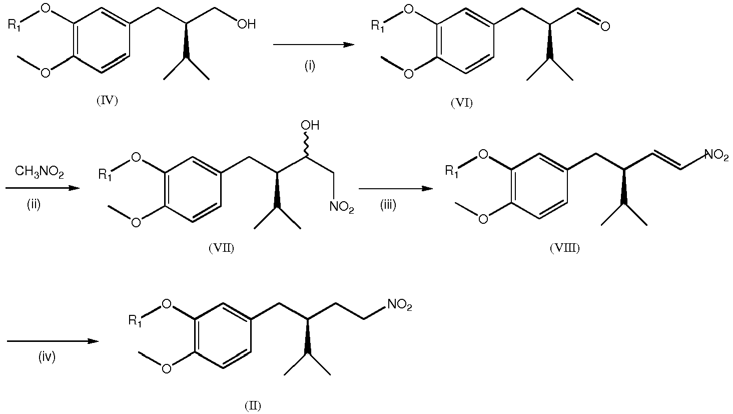

The process involves:
Step (i): oxidation of the primary alcohol of formula IV to give an aldehyde of formula VI;
Step (ii): nitroaldol reaction between the aldehyde substrate VI and nitromethane in the presence of a base, for example a source of fluoride ions, to give the nitro aldol of formula VII;
Step (iii): elimination (dehydration) to give the nitro-alkene of formula VIII;
Step (iv): reduction of the conjugated double bond to give the nitro-alkane of formula II.

Thus, in a preferred practical embodiment of the invention, the primary alcohol of formula IV is subjected to oxidation, for example, by applying a protocol of Swern type. Thus, to a solution of oxalyl chloride and DMSO in dichloromethane (DCM) at -78°C is added dropwise a solution of intermediate IV in DCM, followed after 1 hour by triethylamine (TEA). The reaction mixture is maintained at 0°C for 2 hours and then quenched by addition of saturated NH₄Cl. The organic phases (in DCM) are dried and concentrated to give the aldehyde of formula VI in a final yield of 96-99%. In step (ii), the aldehyde of formula VI is reacted with an excess of nitromethane in the presence of tetrabutylammonium fluoride (TBAF) in THF at 0°C. After about 2 hours, the reaction mixture is quenched by addition of water and the mixture is extracted with ethyl acetate. The organic phases are washed with saturated NaHCO₃, dried and concentrated to give the nitro-aldol product of formula VII in a yield of about 92%. In step (iii), the nitro aldol of formula VII is treated with trifluoroacetic anhydride (TFAA) followed by TEA in DCM at 0°C. After a few hours at 20°C, the reaction mixture is quenched with water and re-extracted with DCM. The organic phases are washed with saturated NH₄Cl, dried and concentrated to give the dehydrated nitro-alkene product of formula VIII in a final yield of 82-89%. In a preferred alternative, steps (ii) and (iii) are performed one-pot by reacting the aldehyde of formula VI with an excess of nitromethane in the presence of ammonium acetate (NH₄OAc) and subsequently refluxing the reaction mixture. In the final step (iv), the nitro-alkene of formula VIII is reduced by treatment with sodium borohydride (NaBH₄) in ethanol at 0°C. After 1-2 hours at 20°C, the reaction mixture is quenched by addition of HCl (1N) and re-extracted with ethyl acetate. The organic phases are concentrated and the crude product is purified by flash chromatography (silica gel) to give the nitro-alkane final product of formula II in a yield of 84-94%.

The compounds of formula II in which R₂ is carboxy, cyano, alkoxy-carbonyl may still be prepared from the hydroxyalkyl derivative of formula IV by converting the latter into the corresponding halo derivative of formula V (X = Cl, Br). This intermediate can react with a nitro derivative of formula 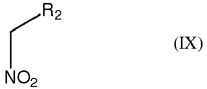 wherein R₂ is defined above; in the presence of a basic species, for example, a source of fluoride ions such as tetrabutylammonium fluoride (TBAF) or KF.

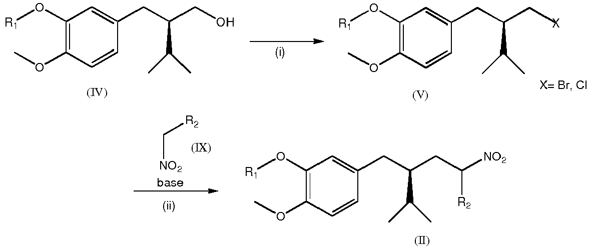

Alternatively, the compounds of formula II in which R₂ is carboxy, alkoxy-carbonyl may still be prepared from the hydroxyalkyl derivative of formula IV by converting the latter into the corresponding aldehyde of formula VI. This intermediate can react with a nitro derivative of formula (IX) in the presence of a basic species, for example a source of fluoride ions, to give a nitro-aldol of formula (XVI), which, by removal of water (dehydration), gives the corresponding nitro-alkene of formula (XVII), which may eventually be converted into the desired product via reduction of the conjugated double bond.

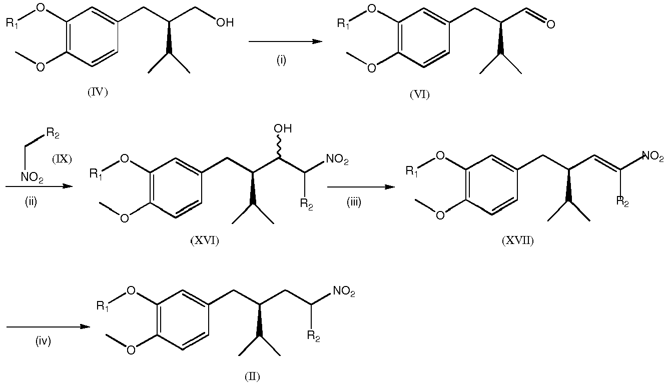

Thus, a further object of the present invention is a process for synthesizing a compound of formula II 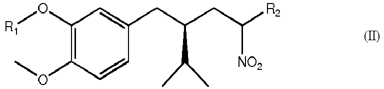 wherein R₁ is a (C₁-C₄)-alkoxy-(C₁-C₄)-alkyl group and R₂ is hydrogen, carboxy, cyano and alkoxy-carbonyl; which comprises
Step (i): oxidizing a primary alcohol of formula IV 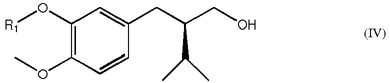 wherein R₁ is defined above, to give an aldehyde of formula VI 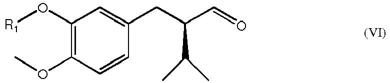 wherein R₁ is defined above;
Step (ii): nitro-aldol reacting an aldehyde substrate VI and a nitro derivative of formula IX 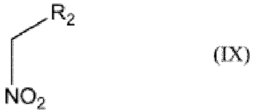 wherein R₂ is defined above; in the presence of a basic species, to give the nitro aldol of formula VII; 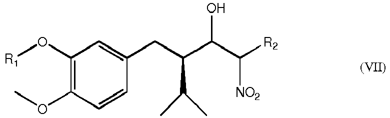 wherein R₁ and R₂ are defined above;
Step (iii): elimination to give a nitroalkene of formula VIII; 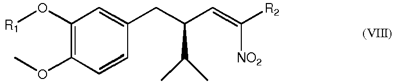 wherein R₁ and R₂ are defined above; and
Step (iv): reducting the conjugated double bond to give a nitro-alkane of formula II. It is a further object of the present invention a compound of formula 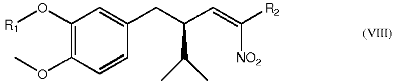 wherein R₁ is a (C₁-C₄)-alkoxy-(C₁-C₄)-alkyl group and R₂ is hydrogen, carboxy, cyano and alkoxy-carbonyl.

It is a further object of the present invention a compound of formula 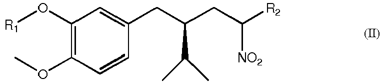 wherein R₁ is a (C₁-C₄)-alkoxy-(C₁-C₄)-alkyl group and R₂ is carboxy, cyano and alkoxy-carbonyl.

The compounds to be considered as preferred are those of formula VIII in which R₁ is a methoxypropyl group and R₂ is hydrogen, such as the compounds of formula II in which R₁ is methoxypropyl.

Said compounds of formulae II and VIII constitute a further object of the present invention as key intermediates in the preparation of 8-aryloctanoic acids having the configuration 2S,4S,5S,7S, in particular aliskiren. Said intermediates in which R₂ is other than hydrogen promote the nitro-aldol reaction by further activating the position on the carbon in α to the nitro group in the deprotection reaction to generate the nucleophilic species which is to be reacted with the aldehyde substrate (electrophile) of formula III.

Compound of formula III in racemic form, for example, may be prepared by ozonolysis of the corresponding 3,4-unsaturated derivative as reported in US 4,409,238.

International patent applications WO 2009/076408 and WO 2009/080773, mentioned above, and WO 2010/010165 describe some possible synthetic approaches to give the compound of formula III both in racemic and in optically active form.

In a preferred aspect of the invention, the compound of formula III in optically active form may be prepared by ozonolysis reaction applied to the substrate of formula Xa or, alternatively, to the substrate of formula Xb according to the scheme reported hereinbelow: 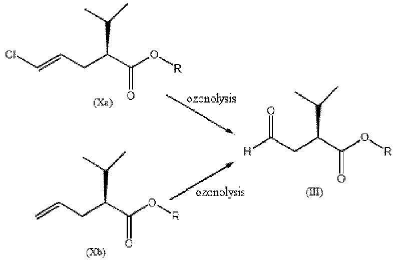 wherein R is hydrogen, benzyl and an optionally branched (C₁-C₆)-alkyl group.

In particular, in a preferred aspect of the invention, the compound of formula III in which R is methyl is synthesized by ozonolysis from the corresponding substrate of formula Xa.

The reaction of a compound of formula II with a compound of formula III to give a compound of formula Ia and/or Ib is, generically, identifiable as a nitro-aldol or Henry reaction between a nucleophile generated from a suitable nitroalkane of formula II and an electrophile consisting of a suitable carbonyl compound III. The product of this reaction is a nitro aldol compound of formula Ia or the corresponding nitrolactone derivative of formula Ib obtained by intramolecular esterification.

The Henry reaction involves reacting the nitronate anion with a carbonyl group of aldehydes and ketones, forming a new C-C bond; nitro aldols (β-hydroxy nitro compounds) are thus obtained, which may optionally be dehydrated to give nitroalkenes (α,β-unsaturated nitro derivatives).

The reaction of a compound of formula II with a compound of formula III to give a compound of formula I is, generally, performed in the presence of a basic species or an organometallic catalyst or, alternatively, a difunctional organic catalyst.

Generally, the reaction object of the invention is performed in organic solvents.

Solvents that are suitable for the coupling reaction of the compounds of formulae II and III are polar aprotic and apolar solvents such as ethers, hydrocarbons and, in particular, acetonitrile, diethoxyethane, 2,2-dimethoxypropane, 1,2-dimethoxyethane, dioxane, toluene and THF, the latter being preferred.

In this case, the substrate is preferably used at a concentration of 1M.

The reaction object of the invention is, generally, performed at a temperature comprised between -10°C and 20°C and, preferably, at 0°C.

The reaction object of the invention is, generally, performed using a nitroalkane of formula II in a mole ratio comprised between 1 and 4 relative to the aldehyde substrate; preferably, the compound of formula II is used in excess relative to the substrate, even more preferably in a mole ratio comprised between 2 and 3 and even more preferably 2.5.

In the reaction object of the invention, the residue R is, preferably, a methyl or ethyl group; R₁ is, preferably, a methoxypropyl group; furthermore, R₂ is, preferably, hydrogen.

In one aspect of the invention, the reaction of a compound of formula II with a compound of formula III to give a compound of formula I is performed in the presence of a basic species.

Suitable bases for the reaction object of the invention are ionic bases such as strong bases that are sources of hydroxyl ion (alkaline hydroxides, for instance NaOH), strong bases that are sources of alkoxide ions (alkaline alkoxides, for instance sodium methoxide), carbonates, for instance sodium carbonate and potassium carbonate and salts that are sources of fluoride ions, for instance tetrabutylammonium fluoride (TBAF) or KF; or non-nucleophilic strong bases of the tertiary amine type (for example DBU), or other basic organic substances (for example guanidine and derivatives).

The reaction was, commonly, performed in the presence of salts that are sources of fluoride ions such as tetrabutylammonium fluoride (TBAF).

The base is, commonly, used in catalytic amount in order to direct the reaction.

Preferably, the base is used relative to the substrate in a mole ratio comprised between 3 and 4.

In a preferred aspect of the invention, said base is added to the reaction mixture consisting of the nitro-derivative, in a variable excess, and the aldehyde in tetrahydrofuran (THF) at 0°C.

In an alternative aspect of the invention, the reaction of a compound of formula II with a compound of formula III to give a compound of formula I is performed in the presence of a catalyst.

The catalyst object of the invention is an organometallic catalyst, among which the preferred ones are organometallic complexes of Cu(II) or Cu(I) with chiral ligands (C2-symmetric or C1-symmetric) derived from optically active diamines such as the known derivatives of chiral 1,2-diaminocyclohexanes or other chiral diamines such as 1,2-diphenylethylenediamine and 2,2'-binaphthyldiamines.

Alternatively, difunctional asymmetric organic catalysts are used in the reaction object of the invention; said catalysts serve to activate both the nucleophile via a basic catalysis and the electrophilic species via the formation of hydrogen bonds, for instance difunctional catalysts containing guanidine-thiourea systems.

The use of a catalyst in the nitro-aldol reaction object of the invention was shown to be surprisingly efficient in terms of yield (about 90%) and, mainly, of stereoselection since it is capable of directing said reaction toward a product of formula I (Ia or Ib) having the desired steric configuration of the chiral centers C(4) and C(5) (4S,5S). It is clear to a person skilled in the art that an enrichment of the diastereoisomeric mixture promotes simplification of the processes for separating the optical isomers that are useful in the preparation of the pharmaceutical active ingredient.

The reaction was, commonly, performed in the presence of an organometallic complex of Cu(II), for instance Cu(OAc)₂ with a chiral ligand, for instance a chiral 1,2-diaminocyclohexane derivative, preferably, the derivative 2-(1,1-dimethylethyl)-6-[[[(1R,2R)-2-[(4-pyridin-yl-methyl)-amino]-cyclohexyl]-amino]-methyl]-phenol. Preferably, the catalyst is used relative to the substrate in an amount comprised between 1 and 10 mol%.

In a preferred aspect of the invention, the nitro derivative, in a variable excess, and the aldehyde in tetrahydrofuran (THF) at 0°C are added to the reaction mixture consisting of said catalyst in solution, optionally, in the presence of a non-nucleophilic aliphatic base. One aspect of the invention envisages that the reaction is performed with a base represented by a source of fluoride ions, for instance tetrabutylammonium fluoride (TBAF) in the presence of THF.

A preferred aspect of the invention envisages that the reaction is performed with a catalyst represented by an organometallic complex of Cu(II) with a chiral ligand, for instance a chiral 1,2-diamino-cyclohexane derivative in the presence of THF.

In the present invention, the term "halogen" means a fluorine, chlorine, bromine or iodine atom.

In an aspect of the invention, the compounds of formulae II and III are dissolved in an ether solvent, preferably, tetrahydrofuran; the solution thus obtained is brought to a temperature of about 0°C and the base, preferably, TBAF is added; the reaction mixture is worked up and the product extracted with a suitable polar solvent, preferably, water making it possible to isolate the compound of formula Ia in good yields (50-65%) as a mixture of four diastereoisomers.

In a preferred aspect of the invention, the ligand and the catalyst are dissolved in an ether solvent, preferably, tetrahydrofuran; the solution thus obtained is brought to a temperature of about 0°C, and a non-nucleophilic aliphatic base, preferably, DIPEA is optionally added; the compounds of formulae II and III, respectively, are then added to said solution; the reaction mixture is worked up, making it possible to isolate the compound of formula Ia in optimal yields (85-90%) as a mixture of the four diastereoisomers, particularly, enriched in the desired isomer (4S,5S).

It is evident to a person skilled in the art that, where a compound of formula III in which R₂ is other than hydrogen is used as substrate, the method object of the invention should envisage the removal of the functional group, generally, performed by decarboxylation.

The product of formula Ia is optionally converted into a compound of formula Ib via known techniques, which involve the intramolecular esterification reaction (lactonization).

This intramolecular lactonization reaction may be obtained by heating the solution of a compound of formula Ia (for example with R = Me) in the presence of an acidic catalysis (for example PTSA). Alternatively, it is possible to obtain the compound of formula Ib in accordance with other methods known in the art not excluding the possible one-pot direct cyclization in the reaction mixture, followed by formation of the nitro aldol ester of formula Ia.

In particular, where R is methyl, the lactonization was performed in refluxing toluene with addition of an acidic catalyst such as p-toluenesulfonic acid (pTSA).

The separation of the optical isomers of formula Ia and/or Ib according to the invention takes place via the use of conventional techniques, such as chromatography, fractional crystallization and other techniques known in the art which exploit the various physicochemical properties of diastereoisomeric compounds.

The 8-aryloctanoic acids having the 2S,4S,5S,7S desired configuration are used in the continuation of the reaction aimed to obtain the pharmaceutically active ingredients.

In one aspect of the invention, the compounds of general formula Ib in which R₁ is defined above are separated on a chromatographic column using silica gel (flash) as stationary phase and an ethyl acetate/n-hexane mixture (8/2) as mobile phase.

In this manner, the diastereoisomers named A, B and a product C that is a mixture of another two diastereoisomers (C1 + C2) are obtained.

The three products A, B, and C of general formula Ib are used separately in the following steps according to the scheme reported below

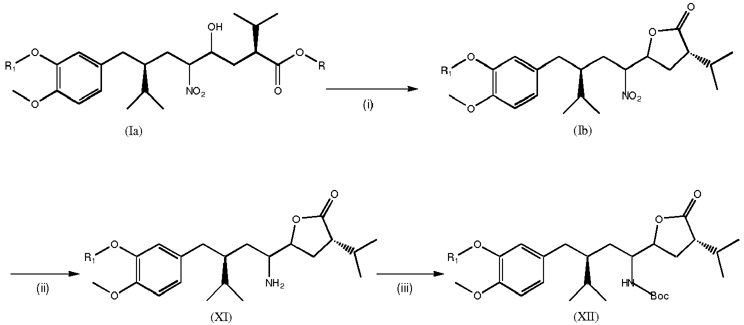

In particular, the compound of formula Ia is converted into the nitro-lactone of formula Ib, which is in turn reduced at the nitro functional group to form the amine group in position 5 and, optionally, protected according to common synthetic techniques.

The reduction of the nitro group in position 5 to an amine according to the invention may consist of a step of catalytic hydrogenation which involves treating the nitro aldol with molecular hydrogen in the presence of a suitable catalyst (e.g. Pd/C), in a protic solvent (e.g. MeOH) with optional addition of acetic acid.

Preferably, in the example of the invention in which the substrate is the nitrolactone of formula Ib, the chemoselective reduction of the nitro group to an amine is obtained by treatment with an excess of trichlorosilane (HSiCl₃, 3.5 eq.) in the presence of a tertiary amine (e.g. DIPEA, 5 eq.) in anhydrous dichloromethane (DCM) at 0°C.

The protection of the amine group takes place according to known techniques.

In this specific case, the Boc group is the elected choice.

The three nitrolactone derivatives A, B, and C of general formula Ib are converted into the corresponding Boc-amino lactone derivatives of general formula XII.

The Boc-amino lactone derivative obtained from the corresponding nitrolactone intermediate C of general formula Ib is further separated, for example, by chromatography into its two isomers named C1 and C2.

Thus, a further object of the present invention is a process for preparing Boc-lactones, which comprises the synthesis of a compound of formula Ia or Ib according to that is described above.

The four Boc-amino lactone derivatives A, B, C1 and C2 of general formula XII thus obtained are converted into the corresponding end products of general formula XV via a sequence which comprises an amidation reaction followed by deprotection of the amine group in position 5 according to techniques that are well known to those skilled in the art, in accordance with the scheme reported below:

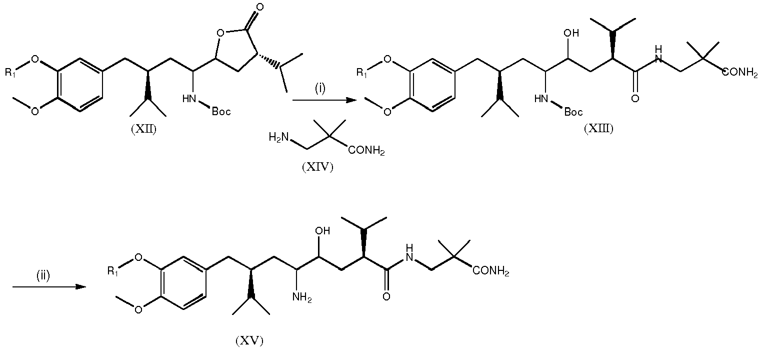

In particular, the coupling reaction with an amino amide derivative of formula XIV may be performed according to techniques known in the art; in particular, the protocol commonly described on substrates of this type involves treating the lactone derivative of formula XII with the amine amide derivative of formula XIV in the presence of 2-hydroxy-pyridine (2-OH-Py) and triethylamine (TEA) either neat or dissolved in tert-butyl methyl ether (TBME) at a temperature of at least 90°C.

The deprotection of the amine group in position 5 takes place according to known techniques to give the free amine function, which is in turn optionally converted into the pharmaceutically acceptable salt, the hemifumarate being preferred, via known techniques. In particular, the synthetic sequence reported above applied to the Boc-amino lactone derivative of formula XII identified as a separate isomer C2 (Boc-lactone) gives rise to a final product of general formula XV having the 2S,4S,5S,7S desired configuration.

Said product constitutes the pharmaceutically active ingredient known as aliskiren (as the hemifumarate salt), which is reported hereinbelow with the molecular structure and the full stereochemistry

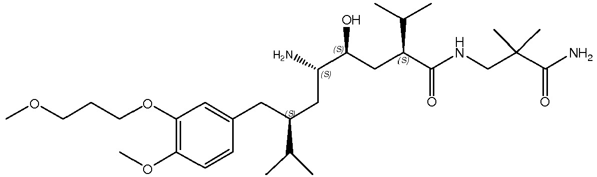

Thus, it is a further object of the present invention a process for preparing aliskiren which comprises the synthesis of a compound of formula Ia or Ib as described above.

The process according to the invention proposes to simplify the preparation of 8-aryloctanoic acids known as antihypertensive agents that interfere with the renin-angiotensin system.

In particular, the present invention relates to an improved method for synthesizing a β-amino alcohol or a lactone analog thereof (Boc-lactone) via the corresponding nitro derivatives of formula Ia and/or Ib.

The present invention is characterized by the reaction of a compound of formula II with a compound of formula III to give a nitro aldol compound of formula Ia or the corresponding nitrolactone derivative of formula Ib obtained by intramolecular esterification.

To the best of the inventors' knowledge, said nitro-aldol reaction or Henry reaction between a nucleophile generated from a suitable nitroalkane of formula II and an electrophile consisting of a suitable carbonyl compound III according to the invention has never been described or suggested in the prior art.

In particular, the prior art does not describe or suggest both the application of said reaction to the substrates object of the invention and the method for preparing the specific compound of formula II as described and claimed herein.

Thus, the present invention is capable of providing, firstly, a chiral synthon (compound II) containing a nitrogen atom (nitro group) in a simple and efficient manner; in addition, said chiral synthon, via the nitro-aldol reaction described above, allows the direct introduction of the optionally protected amine group into position 5 following reduction.

The resolution of the diastereoisomeric mixture according to known techniques leads to the desired optical isomer. It should be noted that this resolution is particularly favored for diastereoisomeric mixtures enriched in said desired optical isomer (4S;5S) obtained by performing the nitro-aldol reaction according to the invention in the presence of a catalyst defined above.

The picture is completed by high yields, an appreciable reduction in the production costs and the production of a product with an optical and chemical purity such that it can be directly subjected to the subsequent phases of the process.

It is thus evident that the preparation method object of the invention constitutes an efficient and economic synthetic alternative in the preparation of key intermediates in the preparation of pharmaceutical active ingredients; in addition, the ready availability of the starting materials used combined with the reduced number of synthetic steps and the good recorded yields definitely entail appreciable advantages in terms of production costs and efficiency. A practical embodiment of the process object of the present invention comprises the optional preparation of a nitro derivative of formula II; the reaction of said nitro derivative with a suitable compound of formula III under the Henry conditions to give a compound of formula Ia or a lactone derivative thereof of formula Ib optionally following conversion; the reduction of the nitro group of a compound of formula Ib and the subsequent protection lead to the key intermediate in the preparation of 8-aryloctanoic acids with renin-inhibiting action.

A preferred practical embodiment of the process object of the present invention comprises the preparation of a nitro derivative of formula II via the corresponding aldehyde derivative; the reaction of said nitro derivative with a suitable compound of formula III under the Henry conditions to give a compound of formula Ia which is appropriately converted into a lactone derivative thereof of formula Ib; the reduction of the nitro group of a compound of formula Ib and the subsequent protection lead to the key Boc-lactone intermediate, which is converted into aliskiren according to known techniques.

For better illustrating the invention the following examples are now given.

### Example 1

### Synthesis of (S)-4-(2-isopropyl-4-nitrobutyl)-1-methoxy-2-(3-methoxypropoxy)-benzene (II).

**Step (i):** synthesis of (αR)-4-methoxy-3-(3-methoxypropoxy)-α-(1-methylethyl)-benzene propanal (VI); anhydrous DMSO (170 µl, 2.39 mmol) was added to a solution of oxalyl chloride (105 µl, 1.187 mmol) in 2 ml of anhydrous DCM, under N₂ and at -78°C. After 15 minutes, a solution of (βR)-4-methoxy-3-(3-methoxypropoxy)-β-(1-methylethyl)-benzene propanol (IV) (112.3 mg, 0.379 mmol) was added dropwise. The mixture was stirred at - 78°C for 1 hour and 350 µl (2.51 mmol) of anhydrous TEA were then added. The mixture was stirred at 0°C for 2 hours. The reaction was monitored by TLC (6/4 HEX/EA) and neutralized with saturated NH₄Cl solution (10 ml); the mixture was extracted with DCM (3 × 10 ml) and the organic phase was washed with brine (10 ml), dried (Na₂SO₄), filtered and evaporated to give 107.3 mg of product (VI) (yield = 96%).
1H NMR (400 MHz, CDCl₃) δ: 9.68 (d, J = 2.8 Hz, 1H), 6.77 (d, J = 8.1 Hz, 1H), 6.71 (d, J = 2.0 Hz, 1H), 6.69 (dd, J = 8.1 and 2.0 Hz, 1H), 4.09 (t, J = 6.5 Hz, 2H), 3.82 (s, 3H), 3.57 (t, J = 6.2 Hz, 2H), 3.36 (s, 3H), 2.92 (dd, J = 14.2 and 9.2 Hz, 1H), 2.70 (dd, J = 14.2 and 5.0 Hz, 1H), 2.50-2.44 (m, 1H), 2.14-1.99 (m, 3H), 1.04 (d, J= 4.4 Hz, 3H), 1.03 (d, J = 4.4 Hz, 3H).
13C NMR (100 MHz, CDCl₃) δ: 205.2, 148.4, 147.9, 132.1, 121.0, 114.1, 111.9, 69.3, 66.0, 59.7, 58.6, 56.0, 31.7, 29.6, 28.3, 19.9, 19.8.
IR (film): 2950.3, 2931.9, 2874.3, 2834.0, 2723.3, 1722.3, 1607.0, 1590.0, 1515.9, 1464.9, 1442.9, 1261.2, 1236.8, 1140.1, 1120.5, 1027.5 cm⁻¹.

**Step (ii):** synthesis of (R)-3-(4-methoxy-3-(3-methoxypropoxy)-benzyl)-4-methyl-1-nitropentan-2-ol (VII):
**1.** CH₃NO₂ (925 ml, 17.2 mmol) was added to a solution of intermediate (VI) (535 mg, 1.725 mmol) in 6.5 ml of anhydrous THF under N₂ at 0°C; next, 1M TBAF in THF was added dropwise (5.8 ml, 5.8 mmol). The solution was stirred for 2 hours at 0°C (the reaction was monitored by TLC, 6/4 HEX/EA) and then neutralized with 15 ml of H₂O.

The mixture was extracted with EA (3 × 10 ml) and the organic phase was washed with saturated NaHCO₃ solution (10 ml) and brine (10 ml), dried (Na₂SO₄), filtered and evaporated to give 560 mg of product (VII) (yield = 92%).

**1 bis:** ammonium acetate (801.65 mg, 10.411 mmol) was added to a solution of intermediate (VI) (2782.6 mg, 9.465 mmol) and CH₃NO₂ (63.112 mg, 10.34 mmol) with stirring. The solution was then brought to reflux and maintained at this temperature for 24 hours. Next, the reaction mixture was cooled, dissolved in DCM/water in a 1/1 ratio (100 ml). The organic phases were extracted with DCM (3 × 50 ml), washed with brine, dried (Na₂SO₄), filtered and concentrated under vacuum to give 2726.1 mg of product (VII) (yield = 85%) as a yellow-orange oil.
1H NMR (300 MHz, CDCl₃) δ: 6.80 (d, J = 9.0 Hz, 1H), 6.78-6.70 (m, 2H), 4.53-4.49 (m, 1H), 4.32 (bs, 1H), 4.20 (dd, J = 6.0 Hz, 2.3 Hz, 1H), 4.18-4.14 (m, 1H), 4.12 (t, J = 6.1 Hz, 2H), 3.86 (s, 3H), 3.59 (t, J = 6.1 Hz, 2H), 3.37 (s, 3H), 2.71 (d, J = 6.2 Hz, 2H), 2.19-2.09 (m, 2H), 1.88-1.84 (m, 1H), 1.68-1.54 (m, 1H), 1.08 (d, J= 3.4 Hz, 3H), 1.01 (d, J = 3.1 Hz, 3H).

**Step (iii):** synthesis of (R,E)-4-(2-isopropyl-4-nitrobuten-3-yl)-1-methoxy-2-(3-methoxypropoxy)-benzene (VIII); 240 µl of distilled TFAA (1.70 mmol) were added to a solution of intermediate (VII) (550 mg, 1.55 mmol) in 5 ml of anhydrous DCM, under N₂ at 0°C, followed by addition of anhydrous TEA (430 µl, 3.1 mmol). The solution was stirred at RT (20°C) for 8 hours and, at 0°C, 240 µl of distilled TFAA (1.70 mmol) were then added, followed by addition of anhydrous TEA (430 µl, 3.1 mmol). The solution was stirred at RT (20°C) for 12 hours, diluted with 10 ml of DCM and mixed with 10 ml of H₂O; the organic phases were then separated out and the aqueous phase was extracted with DCM (10 × 2 ml). All the organic phases were washed with saturated NH₄Cl solution (10 ml) and brine (10 ml), dried (Na₂SO₄), filtered and evaporated. The crude product was purified by chromatography (8/2 → 7/3 HEX/EA) to give 428.2 mg of product (VIII) (yield = 82%).
1H NMR (300 MHz, CDCl₃) δ: 7.15 (dd, J = 10.0 and 2.0 Hz, 1H), 6.76 (d, J = 7.0 Hz, 1H), 6.68 (d, J = 10.0 Hz, 1H), 6.67-6.59 (m, 2H), 4.13 (t, J = 6.0 Hz, 2H), 3.86 (s, 3H), 3.55 (t, J = 6.1 Hz, 2H), 3.35 (s, 3H), 2.86 (dd, J = 15 and 6.0 Hz, 1H), 2.57 (dd, J = 15 and 9.0 Hz, 1H), 2.39-2.27 (m, 1H), 2.16-2.04 (m, 2H), 1.86-1.82 (m, 1H), 1.68-1.54 (m, 1H), 0.99 (d, J= 5.5 Hz, 3H), 0.95 (d, J = 5.1 Hz, 3H).

**Step (iv):** synthesis of (S)-4-(2-isopropyl-4-nitrobutyl)-1-methoxy-2-(3-methoxypropoxy)-benzene (II): NaBH₄ was added to a solution of intermediate (VIII) (1.46 mmol) in 4 ml of EtOH, at 0°C; the reaction was stirred at RT for 1 hour 30 minutes. 4 ml of 1N HCl were added at 0°C and the mixture was extracted with EA (5 × 3 ml). The organic phase was dried (Na₂SO₄, filtered and evaporated. The crude product was purified by flash chromatography (7/3 HEX/EA) to give 415 mg of product (II) (yield = 84%).
1H NMR (300 MHz, CDCl₃) δ: 6.81 (d, J = 6.0 Hz, 1H), 6.72-6.68 (m, 2H), 4.22 (t, J= 6.1 Hz, 2H), 4.12 (t, J= 5.5 Hz, 2H), 3.86 (s, 3H), 3.60 (t, J= 5.5 Hz, 2H), 3.38 (s, 3H), 2.68 (dd, J= 12.0 and 6.2 Hz, 1H), 2.35 (dd, J= 12.0 and 9.2 Hz, 1H), 2.14-2.05 (m, 3H), 1.88-1.75 (m, 1H), 1.75-1.70 (m, 1H), 1.68-1.55 (m, 1H), 0.97 (d, J=5.1Hz, 3H), 0.93 (d, J= 5.7 Hz, 3H). 13C NMR (100 MHz, CDCl₃) δ: 148.5, 147.2, 133.1, 122.0, 114.1, 112.7, 75.3, 67.0, 65.7, 58.5, 56.2, 43.1, 35.7, 29.8, 29.6, 28.0, 19.1, 18.8.
HRMS C₁₈H₂₉NO₅ [M + Na+] expected 362.1944, found 362.19348.

### Example 2

### Synthesis of methyl (S)-isopropyl-4-oxo butanoate (III)

265 ml of toluene and 66 ml of 2-propanol were added to 33 g (0.173 mol) of (S)-E-methyl-5-chloro-2-isopent-4-enoate (Xa) and the pH was adjusted to between 6 and 9 with 0.5 ml of triethylamine. The solution was cooled to -50°C and ozone was then introduced into the reaction mixture while maintaining the temperature at -50°C. The pH was adjusted to between 1 and 10 by addition of triethylamine until the reaction was complete. 22.5 g (0.182 mol) of trimethyl phosphite were added at -50°C and the reaction mixture was then warmed to 20°C. Water was added and the aqueous and organic phases were then separated. The aqueous phase was re-extracted with toluene.

The combined organic fractions were washed twice with water and then with aqueous NaHCO₃. The organic (toluene) fractions were concentrated under vacuum. The crude product thus obtained was purified by column chromatography using as eluent a mixture of n-heptane/ethyl acetate, thus, giving the desired product methyl (S)-2-isopropyl-4-oxo butanoate (III) as a yellow oil (molar yield = 70%).
1H NMR (400 MHz, CDCl₃) δ: 9.79 (s, 1H), 3.60 (s, 3H), 2.90-2.75 (m, 1H), 2.70-2.65 (m, 1H), 2.45-2.35 (dd, 1H), 2.00-1.85 (m, 1H), 0.95 (dd, 6H).

### Example 3

### Synthesis of (2S,7S)-methyl 4-hydroxy-2-isopropyl-7-(4-methoxy-3-(3-methoxy propoxy)-benzyl)-8-methyl-5-nitronanoate.

Mixture of diastereoisomers at the two stereocenters C4 and C5; 3.4 eq. of TBAF at 0°C were added to a mixture of 1.1 eq. of (S)-4-(2-isopropyl-4-nitrobutyl)-1-methoxy-2-(3-methoxypropoxy)-benzene (II) and 1 eq. of methyl (S)-2-isopropyl-4-oxo butanoate (III) in a 1M solution in THF. After 90 minutes, the aldehyde appeared to have all been consumed, ethyl acetate was added and the mixture was extracted with H₂O. The organic phases were washed with saturated NaHCO₃ and brine. The product was purified by column chromatography with 7/3 hexane/ethyl acetate to give product (Ia) as a mixture of diastereoisomers (yield = 55%).
1H NMR (300 MHz, CDCl₃) δ: 6.80 (d, J = 9.0 Hz, 1H), 6.72 (d, J = 9.0 Hz, 1H), 6.70-6.64 (m, 1H), 4.50 and 4.15 (m, 1H), 4.14 (t, J= 6.5 Hz, 2H), 3.85 (s, 3H), 3.70 (s, 3H), 3.60 (t, J= 6.0 Hz, 2H), 3.37 (s, 3H), 2.75-2.55 (m, 1H), 2.55-2.41 (m, 2H), 2.24-2.10 (m, 3H), 1.95-1.758 (m, 1H), 1.75-1.70 (m, 1H), 1.30-1.20 (m, 1H), 0.99-0.80 (m, 15 H).

### Example 4

### Synthesis of (3S)-3-isopropyl-5-((3S)-3-(4-methoxy-3-(3-methoxypropoxy)-benzyl)-4-methyl-1-nitropentyl)-dihydrofuran-2(3H)-one (Ib).

Mixture of diastereoisomers; 950 mg of nitro ester (Ia) (1.9 mmol) were dissolved in 10 ml of toluene in a 50 ml round-bottomed flask equipped with a condenser and a CaCl₂ guard tube. 4 Å MS molecular sieves were then added, followed by 32 mg of PTSA (0.19 mmol).

The reaction was refluxed with stirring for 20 hours. The reaction was monitored on TLC plates with 7/3 hexane/EtOAc (Rf = 0.33, similar to the starting material) on which the various stereoisomers were able to be distinguished. The reaction was left to cool to room temperature and the toluene was then evaporated off under vacuum to give the crude nitrolactone product (Ib) as a mixture of diastereoisomers.

Separation of the diastereoisomers: the purification was performed in a chromatography column 4 cm in diameter and 18 cm long using flash silica as stationary phase and an 8/2 HEX/EtOAc mixture as mobile phase. By collecting 75 ml fractions, 3 different diastereoisomers were successfully separated.
Diastereoisomer A present in fractions 15-16-17 = 165 mg;
Diastereoisomer B present in fractions 19/22 = 101 mg;
Product C present in fractions from 24 to 30 = 435 mg;
This product in reality proves to be a mixture of two diastereoisomers (C1 and C2), one of which was the desired diastereoisomer with four stereocenters of (S) absolute configuration. Total yield for the products isolated by chromatography = 79%.
Isomer A: 1H NMR (300 MHz, CDCl₃) δ: 6.81 (d, J = 6.0 Hz, 1H), 6.66-6.64 (m, 2H), 4.55-4-50 (m, 2H), 4.12 (t, J= 6.1 Hz, 2H), 3.86 (s, 3H), 3.62 (t, J= 6.0 Hz, 2H), 3.37 (s, 3H), 2.75 (dd, J= 15 and 6.0 Hz, 1H), 2.57-2.52 (m, 1H), 2.20-2.05 (m, 6H), 2.00-1.60 (m, 3H), 1.70-1.55 (m, 1H), 1.05-0.98 (m, 6 H), 0.96-0.86 (m, 6 H).
Isomer B: 1H NMR (300 MHz, CDCl₃) δ: 6.79 (d, J = 7.0 Hz, 1H), 6.73-6.64 (m, 2H), 4.55-4.40 (m, 1H), 4.14-4.10 (m, 3H), 3.86 (s, 3H), 3.61 (t, J= 6.0 Hz, 2H), 3.36 (s, 3H), 2.75-2.45 (m, 2H), 2.22-2.05 (m, 6H), 1.95-1.60 (m, 3H), 1.70-1.55 (m, 1H), 1.05-0.86 (m, 12 H).
Isomer C: 1H NMR (300 MHz, CDCl₃) δ: 6.81 (d, J = 8.0 Hz, 1H), 6.75-6.68 (m, 2H), 4.63-4-56 (m, 1H), 4.14 (t, J= 6.1 Hz, 2H), 4.14-4.11 (m, 1H), 3.86 (s, 3H), 3.60 (t, J= 6.0 Hz, 2H), 3.37 (s, 3H), 2.75 (dd, J= 17 and 6.0 Hz, 1H), 2.50-2.34 (m, 2H), 2.18-2.07 (m, 5H), 1.80-1.60 (m, 2H), 1.55-1.40 (m, 2H), 0.99 (d, J= 9.0 Hz, 3H), 0.91-0,85 (m, 9 H).

These diastereoisomers were used separately in all the subsequent reaction steps.

### Example 5

### Synthesis of (S)-5-((S)-1-amino-3-(4-methoxy-3-(3-methoxypropoxy)-benzyl)-4-methylpentyl)-3-isopropyl-dihydrofuran-2(3H)-one (XI).

Single diastereoisomer A (stereochemistry defined on 2/4 of the stereocenters): 101 mg of nitrolactone (Ib) diastereoisomer A (0.22 mmol) were dissolved in 0.5 ml of anhydrous DCM in a round-bottomed flask under a N₂ atmosphere. 189 µl of DIPEA (1.08 mmol) were then added and the mixture was cooled to 0°C. Separately, 770 µl of a 1M solution of HSiCl₃ in anhydrous DCM were prepared and subsequently added to the reaction mixture (the evolution of fumes is noted). The reaction was allowed to return to room temperature and, after 16 hours, was diluted with 20 ml of DCM and transferred into a separating funnel, where it was treated with 3 ml of saturated NaHCO₃ solution (an appreciable amount of silicates was formed, which make the solution turbid). The two phases were separated and the aqueous phase was diluted with 10 ml of H₂O and extracted 3 times with 20 ml of DCM. The organic phases were combined and washed with 10 ml of saturated NaCl solution. The resulting solution was dried with sodium sulfate, filtered and the solvent was removed under vacuum. The amino lactone product (XI) (102 mg) was obtained as a single diastereoisomer A.

Analogously: starting with 95 mg of nitrolactone (Ib) diastereoisomer B, 85 mg of amino lactone (XI) were obtained as a single diastereoisomer B; starting with 355 mg of nitrolactone (Ib), as a mixture of diastereoisomers C, 325 mg of amino lactone (XI) were obtained as a mixture of diastereoisomers C. On TLC in 1/1 HEX/EtOAc, the product does not run and remains on the baseline, and was developed with ninhydrin.

The product was used as crude in the subsequent reaction.

### Example 6

### Synthesis of tert-butyl (S)-1-((S)-4-isopropyl-5-oxo-2-tetrahydrofuranyl)-3-(4-methoxy-3-(3-methoxypropoxy)-benzyl)-4-methylpentyl-carbamate (XII).

Single diastereoisomer A (stereochemistry defined on 2/4 of the stereocenters): the amino lactone intermediate (XI) diastereoisomer A (85 mg; 0.195 mmol) was dissolved in 5 ml of MeOH in a 25 ml round-bottomed flask. 50 mg of Boc₂O (0.23 mmol) were then added and the reaction was left at room temperature for 18 hours.

The reaction mixture was diluted with 40 ml of EtOAc and transferred into a separating funnel where it was washed with 10 ml of saturated NaCl solution. The aqueous phases were taken up (2 × 30 ml) with EtOAc and the combined organic phases were dried with sodium sulfate, filtered on a fluted filter and the solvent was removed under vacuum.

The purification was performed in a chromatography column with a diameter of 1.3 cm and 10 cm tall, collecting 15 ml fractions. Flash silica was used as stationary phase and 98/2 DCM/MeOH was used as eluent. The product was contained in fractions 7→12. The Boc-amino lactone product (XII) (86 mg; 0.16 mmol) was obtained as a single diastereoisomer A. Analogously: starting with 70 mg of amino lactone (XI) diastereoisomer B, 60 mg of Boc-amino lactone (XII) were obtained as a single diastereoisomer B; starting with 330 mg of amino lactone (XI) as a mixture of diastereoisomers C, 227 mg of Boc-amino lactone (XII) were obtained as a mixture of diastereoisomers C (and 90 mg of unreacted amine).

In another preparation, starting with 220 mg of amino lactone (XI) as a mixture of diastereoisomers C, 114 mg of Boc-amino lactone (XII) were obtained as a mixture of diastereoisomers C (mixed isomers) (and 100 mg of unreacted amine).

The two diastereoisomers C1 and C2 of the Boc-amino lactone (XII) were separated by careful column chromatography. The following were recovered: 60 mg of diastereoisomer C1 and 51 mg of diastereoisomer C2.
Isomer A: 1H NMR (300 MHz, CDCl₃) *δ* (ppm) 6.80-6.67 (m, 3H), 4.41-4.27 (m, 2H), 4.09 (t, J =6.0 Hz, 2H), 3.83 (s, 3H), 3.57 (t, J =6.4 Hz, 2H), 3.58-3.47 (m, 1H), 3.34 (s, 3H), 2.58-2.42 (m, 3H), 2.11-1.91 (m, 5H), 1.68-1.30 (m, 2H), 1.47 (s, 9H), 1.30 - 1.2 (m, 2H), 0.91 (d, J =6.1 Hz, 3H), 0.87 (d, J =6.4 Hz, 3H), 0.85 (d, J =7.8 Hz, 6H);
HRMS C₃₀H₄₉NO₇ [M + Na+] calcd 558.3407, found 558.3401.
Isomer B: 1H NMR (300 MHz, CDCl₃) *δ* (ppm) 6.85-6.67 (m, 3H), 4.45-4.22 (m, 2H), 4.11 (t, J =6.0 Hz, 2H), 3.83 (s, 3H), 3.62-3.57 (m, 1H), 3.55 (t, J =6.4 Hz, 2H), 3.36 (s, 3H), 2.70-2.33 (m, 3H), 2.21-2.03 (m, 5H), 1.68-1.53 (m, 3H), 1.50 (s, 9H), 1.30 - 1.2 (m, 1H), 1.01 (d, J =6.1 Hz, 3H), 0.86 (d, J =6.4 Hz, 3H), 0.82 (d, J =7.8 Hz, 6H);
HRMS C₃₀H₄₉NO₇ [M + Na+] calcd 558.3407, found 558.3410.
Isomer C1: 1H NMR (300 MHz, CDCl₃) *δ* (ppm) 6.75-6.65 (m, 3H), 4.35-4.22 (m, 2H), 4.10 (t, J =6.0 Hz, 2H), 3.83 (s, 3H), 3.77-3.65 (m, 1H), 3.56 (t, J =6.4 Hz, 2H), 3.36 (s, 3H), 2.63-2.38 (m, 3H), 2.19-2.03 (m, 5H), 1.71-1.43 (m, 3H), 1.45 (s, 9H), 1.30 - 1.2 (m, 1H), 0.99 (d, J =5.5 Hz, 3H), 0.98-0.85 (m, 9H).
HRMS C₃₀H₄₉NO₇ [M + Na+] calcd 558.3407, found 558.3410.
Isomer C2: 1H NMR (300 MHz, CDCl₃) *δ* (ppm) 6.77 (d, J=8.0 Hz, 1H), 6.76-6.69 (m, 2H), 4.41-4.37 (m, 2H), 4.11 (t, J =6.5 Hz, 2H), 3.84 (s, 3H), 3.85-3.75 (m, 1H), 3.57 (t, J =6.4 Hz, 2H), 3.37 (s, 3H), 2.60 (dd, J=12.0, 5.5 Hz, 1H), 2.57-2.52 (m, 1H), 2.40 (dd, J= 14.0, 9.0 Hz, 1H), 2.20-2.01 (m, 5H), 1.68-1.30 (m, 3H), 1.47 (s, 9H), 1.32- 1.2 (m, 1H), 1.01 (d, J =6.1 Hz, 3H), 0.95 (d, J =6.4 Hz, 3H), 0.84 (d, J =7.8 Hz, 6H);
13C NMR (75 MHz, CDC13) *δ* (ppm) 179.2, 156.4, 148.4, 147.8, 133.9, 121.5, 114.5, 111.9, 81.6, 79.9, 69.6, 66.2, 58.8, 56.2, 52.1, 46.0, 42.6, 37.6, 33.5, 29.8, 29.4, 28.5 (3C), 27.9, 26.8, 20.6, 20.5, 18.6, 16.7.
HRMS C₃₀H₄₉NO₇ [M + Na+] calcd 558.3407, found 558.3403.

### Example 7

### Synthesis of tert-butyl (3S,8S)-8-(3-amino-2,2-dimethyl-3-oxopropyl-carbamo-yl)-6-hydroxy-3-(4-methoxy-3-(3-methoxypropoxy)-benzyl)-2,9-dimethyldecan-5-yl-carbamate (XIII).

Single diastereoisomer A (stereochemistry defined on 2/4 of the stereocenters): the Boc-amino lactone (XII) diastereoisomer A (60 mg; 0.112 mmol) was dissolved in 1 ml of methyl tert-butyl ether and 100 µl of TEA in a round-bottomed flask equipped with a condenser and a CaCl₂ guard tube. 19.5 mg of 3-amino-2,2-dimethyl propionamide (XIV) (0.17 mmol) and 12.5 mg of 2-OH-Py (0.132 mmol) were then added. The reaction proceeds at 90°C for 72 hours. The mixture was allowed to cool to room temperature, diluted with 10 ml of toluene and treated with 10 ml of aqueous 10% KHSO₄ solution. The aqueous phases were taken up in 7 ml of toluene and the combined organic phases were washed with 5 ml of saturated NaCl solution. The resulting solution was dried with sodium sulfate and filtered through a fluted filter and the solvent was removed under vacuum. The product was purified on a chromatography column 1 cm in diameter and 10 cm tall, collecting 15 ml fractions. Flash silica was used as stationary phase and 200 ml of 97/3 DCM/MeOH and 200 ml of 9/1 DCM/MeOH were used as eluent. The product was present in fractions 18, 19, 20. The product Boc-aliskiren (XIII) (38 mg; 0.058 mmol) was obtained as a single diastereoisomer A.

Analogously: starting with 50 mg of Boc-amino lactone (XII) diastereoisomer B, 28 mg of Boc-aliskiren (XIII) were obtained as a single diastereoisomer B; starting with 204 mg of Boc-amino lactone (XII) as a mixture of diastereoisomers C, 105 mg of Boc-aliskiren (XIII) were obtained as a mixture of diastereoisomers C.

By repeating the reaction on separated isomers of Boc-amino lactone (XII) mixture C, the following were obtained: 35 mg of Boc-aliskiren (XIII) diastereoisomer C1 from 50 mg of starting material; and 23 mg of Boc-aliskiren (XIII) diastereoisomer C2 from 40 mg of starting material. TLC: stationary phase silica, mobile phase DCM/MeOH. The product was developed with an oxidizing solution based on cerium and molybdenum salts. Rf = 0.48. Isomer A: 1H NMR (300 MHz, CDCl₃) *δ* (ppm): 6.75-6.65 (m, 3H), 6.42 (m, 1H), 6.25 (m, 1H), 5.68 (m, 1H), 4.70 (m, 1H), 4.09 (t, J =6.1 Hz, 2H), 3.84 (s, 3H), 3.58 (t, J =6.0 Hz, 2H), 3.55-3.45 (m, 3H), 3.36 (s, 3H), 3.33 (m, 1H), 2.62 (dd, J = 4.8, 10.5 Hz, 1H), 2.42 (dd, J = 6.1, 14.5 Hz, 1H), 2.22-2.04 (m, 4H), 1.75 (m, 2H), 1.70-1.40 (m, 5H), 1.40 (s, 9H), 1.22 (m, 1H), 1.14 (m, 6H), 0.89-0.75 (m, 12H).
HRMS C₃₅H₆₁N₃O₈ [M +H+] calcd 652.4492, found 652.4493.
Isomer B: 1H NMR (300 MHz, CDCl₃) *δ* (ppm): 6.80-6.66 (m, 3H), 6.33 (m, 1H), 5.5 (m, 1H), 4.80 (m, 1H), 4.10 (t, J =6.0 Hz, 2H), 3.82 (s, 3H), 3.60 (t, J =6.0 Hz, 2H), 3.55-3.35 (m, 3H), 3.36 (s, 3H), 3.33 (m, 1H), 2.58 (dd, J = 5.0, 12.5 Hz, 1H), 2.42 (m, 1H), 2.22-2.04 (m, 3H), 1.75 (m, 3H), 1.70-1.40 (m, 3H), 1.40 (s, 9H), 1.22 (m, 9H), 0.90-0.82 (m, 12H).
HRMS C₃₅H₆₁N₃O₈ [M +H+] calcd 652.4492, found 652.4490.
Isomer C1: 1H NMR (300 MHz, CDCl₃) *δ* (ppm): 6.75-6.55 (m, 3H), 6.51 (m, 1H), 6.20 (m, 1H), 5.73 (m, 1H), 4.70 (m, 1H), 4.09 (t, J =6.1 Hz, 2H), 3.81 (s, 3H), 3.56 (t, J =6.0 Hz, 2H), 3.48-3.35 (m, 3H), 3.36 (s, 3H), 3.33 (m, 1H), 2.61 (dd, J = 4.1, 12 Hz, 1H), 2.37 (m, 1H), 2.22 (m, 1H), 2.20-2.01 (m, 3H), 1.75 (m, 1H), 1.65-1.50 (m, 4H), 1.41 (s, 9H), 1.20-1.14 (m, 7H), 0.92-0.80 (m, 12H).
HRMS C₃₅H₆₁N₃O₈ [M +H+] calcd 652.4492, found 652.4490.
Isomer C2: 1H NMR (300 MHz, CDCl₃) *δ* (ppm): 6.81-6.65 (m, 3H), 6.51 (m, 1H), 6.25 (m, 1H), 5.55 (m, 1H), 4.50-4-25 (m, 1H), 4.11 (t, J =6.1 Hz, 2H), 3.86 (s, 3H), 3.58 (t, J =6.0 Hz, 2H), 3.35-3.31 (m, 1H), 3.37 (s, 3H), 2.61-2.50 (m, 2H), 2.40 (m, 1H), 2.22 (m, 1H), 2.20-2.01 (m, 3H), 1.75 (m, 1H), 1.65-1.50 (m, 2H), 1.41 (s, 9H), 1.28-1.14 (m, 8H), 0.95-0.80 (m, 12H),
HRMS C₃₅H₆₁N₃O₈ [M +H+] calcd 652.4492, found 652.4491.

### Example 8

### Synthesis of tert-butyl (3S,8S)-8-(3-amino-2,2-dimethyl-3-oxo-propyl-carbamo-yl)-6-hydroxy-3-(4-methoxy-3-(3-methoxypropoxy)-benzyl-2,9-dimethyldecan-5-yl carbamate (aliskiren diastereoisomer A).

Single diastereoisomer A (stereochemistry defined on 2/4 of the stereocenters): 36 mg of Boc-aliskiren (XIII) diastereoisomer A (0.055 mmol) were treated with 1 ml of a 5/1 DCM/TFA solution. The reaction proceeds for 2 hours, after which it was diluted with a further 10 ml of DCM and treated with 30% NaOH to pH ≈ 8-9 (about 1 ml). 2 ml of H₂O were then added and the phases were separated. The aqueous phase was taken up in DCM (2 × 15 ml). The combined organic phases were dried with sodium sulfate and filtered on a fluted filter, and the solvent was removed under vacuum.

The product was observed by developing with ninhydrin: 23 mg of crude product were obtained which was purified by column chromatography (eluent 8/2 DCM/MeOH) to give 16 mg of pure product, Aliskiren diastereoisomer A.

Analogously: starting with 18 mg of Boc-aliskiren (XIII) diastereoisomer B, 11 mg of Aliskiren diastereoisomer B were obtained; starting with 45 mg of Boc-aliskiren (XIII) mixed isomers C, 23 mg of Aliskiren diastereoisomer mixture C were obtained (and 15 mg of starting material). The unblocking yields appear to be improved if, after having performed the deprotection with trifluoroacetic acid, the reaction mixture was treated with solid potassium carbonate rather than aqueous sodium hydroxide.

NOTE: the final product classified as isomer A, isomer B and also the two products defined as isomers C have, as demonstrated by HRMS, the exact mass of the product (Aliskiren). Aliskiren diastereoisomer C2 was identified as being the desired product with the four stereocenters of (S) absolute configuration: tert-butyl (3S,5S,6S,8S)-8-(3-amino-2,2-dimethyl-3-oxo-propyl-carbamole)-6-hydroxy-3-(4-methoxy-3-(3-methoxypropoxy)-benzyl)-2,9-dimethyldecan-5-yl carbamate (Aliskiren).
Isomer A: 1H NMR (300 MHz, CDCl₃) δ 6.82-6.60 (m, 3H), 6.55-6.45 (m, 1H), 6.83 (broads, 1H), 6.22-6.03 (m, 1H), 5.50 (m, 1H), 4.13 (t, J = 5.5 Hz, 2H), 3.87 (s, 3H), 3.68-3.60 (m, 1H), 3.55 (t, J = 6.0 Hz, 2H), 3.33 (s, 3H), 3.26-3.11 (m, 1H), 2.65 (dd, J = 3.1, 9 Hz, 1H), 2.55-2.44 (m, 1H), 2.15-2.03 (m, 3H), 1.90 (broad s, 1H), 1.71-1.52 (m, 8H), 1.24-1.20 (m, 8H), 0.96-0.85 (m, 12H).
HRMS (+ ESI MS) Found 552.40230 [M + H]+, C₃₀H₅₄N₃O₆ requires 552.40071; found 574.40139 [M + Na]+,C₃₀H₅₃NaN₃O₆ requires 574.38321, found 574.38323.
Isomer B: 1H NMR (300 MHz, CDCl₃) δ 6.82-6.60 (m, 3H), 6.55-6.45 (m, 1H), 6.83 (broads, 1H), 6.22-6.03 (m, 1H), 5.50 (m, 1H), 4.13 (t, J = 5.5 Hz, 2H), 3.87 (s, 3H), 3.68-3.60 (m, 1H), 3.55 (t, J = 6.0 Hz, 2H), 3.33 (s, 3H), 3.26-3.11 (m, 1H), 2.65 (dd, J = 3.1, 9 Hz, 1H), 2.55-2.44 (m, 1H), 2.15-2.03 (m, 3H), 1.90 (broad s, 1H), 1.71-1.52 (m, 8H), 1.24-1.20 (m, 8H), 0.96-0.85 (m, 12H).
HRMS (+ ESI MS) Found 552.40230 [M + H]+, C₃₀H₅₄N₃O₆ requires 552.40071; found 574.40092 [M + Na]+,C₃₀H₅₃NaN₃O₆ requires 574.38321, found 574.38327.
Isomer C1: 1H NMR (300 MHz, CDCl₃) δ 6.76-6.68 (m, 3H), 6.50-6.45 (m, 1H), 6.35 (m, 1H), 5.50 (m, 1H), 4.10 (t, J = 5.1 Hz, 2H), 3.90 (m, 1H), 3.82 (s, 3H), 3.57 (t, J = 6.0 Hz, 2H), 3.45-3.31 (m, 1H), 3.32 (s, 3H), 3.1 (m, 1H), 2.75-2.34 (m, 4H), 2.15-2.03 (m, 4H), 1.90-1.50 (m, 6H), 1.30-1.19 (m, 8H), 0.99-0.85 (m, 12H).
HRMS (+ ESI MS) Found 552.40230 [M + H]+, C₃₀H₅₄N₃O₆ requires 552.40071; found 574.40082 [M + Na]+,C₃₀H₅₃NaN₃O₆ requires 574.38321, found 574.38322.
Isomer C2: 1H NMR (300 MHz, CDCl₃) δ 6.80-6.60 (m, 3H), 4.11 (t, J = 6.5 Hz, 2H), 4.10-4.00 (m, 1H), 3.85 (s, 3H), 3.55 (t, J = 6.3 Hz, 2H), 3.33 (s, 3H), 2.85 (m, 1H), 2.75 (m, 1H), 2.60-2.38 (m, 3H), 2.15-2.03 (m, 3H), 1.95-1.60 (m, 5H), 1.49-1.35 (m, 1H), 1.25-1.20 (m, 8H), 0.90-0.81 (m, 12H).
HRMS (+ ESI MS) Found 552.40230 [M + H]+, C₃₀H₅₄N₃O₆ requires 552.40071; found 574.40152 [M + Na]+,C₃₀H₅₃NaN₃O₆ requires 574.38321, found 574.38315.

### Example 9

### Synthesis of (2S,7S)-methyl 4-hydroxy-2-isopropyl-7-(4-methoxy-3-(3-methoxy-propoxy)-benzyl)-8-methyl-5-nitro nonanoate.

Mixture of diastereoisomers at the two stereocenters C4 and C5: the ligand 2-(1,1-dimethylethyl)-6-[[[(1R,2R)-2-[(4-pyridinylmethyl)-amino]-cyclohexyl]-amino]-methyl]-phenol (2.9 mg, 0.0158 mmol) was dissolved in dry THF (1 ml) under an N₂ atmosphere; Cu(OAc)₂ (5.8 mg, 0.158 mmol) was added and the mixture was stirred at room temperature for 30 minutes.

After 10 minutes, DIPEA (4.1 mg, 0.0316 mmol) was added and the reaction mixture was cooled to 0°C; next, the nitroalkane of formula II (321.8 mg, 0.9494 mmol) was added; finally, the aldehyde of formula VIII (50 mg, 0.3165 mmol) was added and the reaction mixture was stirred at 0°C for 48 hours. After a further 48 hours, the solvent was removed under vacuum and the crude product was purified by column chromatography with 85/15 hexane/ethyl acetate to give product (Ia) as a colorless oil as a mixture of diastereoisomers (140.8 mg, yield = 90%) which is particularly enriched in one of them.

### Example 10

### Synthesis of (3S)-3-isopropyl-5-((3S)-3-(4-methoxy-3-(3-methoxypropoxy)-benzyl)-4-methyl-1-nitropentyl)-dihydrofuran-2(3H)-one (Ib).

According to the procedure described in Example 4 above, the compound of formula Ia obtained in Example 9 was converted into the corresponding lactone of formula Ib.

Separation of the diastereoisomers by column chromatography revealed 3 different diastereoisomers.
Diastereoisomer A (2%);
Diastereoisomer B (18%);
Product C (80%).

Said product C, once converted into the Boc-lactone derivative of general formula XII according to the procedures described in Examples 5 and 6 above, proved to be a single predominant diastereoisomer of the two possible ones (C1 and C2).

Specifically, the process according to the invention leads to the Boc-lactone diastereoisomer C2 bearing the 4 stereocenters with the (S) desired absolute configuration.

It is thus evident to a person skilled in the art that the process according to the invention, besides having very high yields, makes it possible to simply and efficiently obtain a key synthon (lactone) in the synthesis of a pharmaceutical active principle, in particular aliskiren, bearing the correct steric configuration at all four of the chiral centers present in the molecular structure.

## Claims

1. A process for preparing a compound of formula 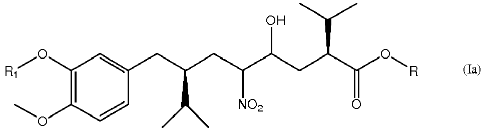 wherein R is hydrogen, benzyl and an optionally branched (C₁-C₆)-alkyl group and R₁ is a (C₁-C₄)-alkoxy-(C₁-C₄)-alkyl group; or a lactone derivative thereof of formula 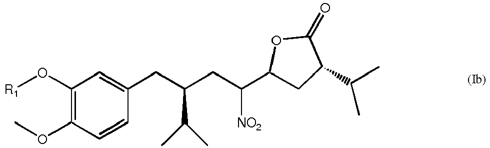 wherein R₁ is defined above which comprises
a. reacting a compound of formula 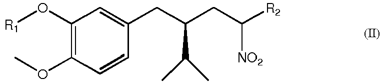 wherein R₁ is defined above and R₂ is hydrogen, carboxy, cyano and alkoxy-carbonyl; with a compound of formula 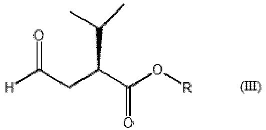 wherein R is defined above; in the presence of a basic species or a catalyst;
b. optionally lactonizing a compound of formula Ia into a compound of formula Ib.

2. A process according to claim 1 wherein said basic species is selected among alkaline hydroxides, alkaline alkoxides, carbonates and salts source of fluoride ions.

3. A process according to claim 2 wherein said basic species is tetrabutylammonium fluoride.

4. A process according to claim 1 wherein said catalyst is selected among an organometallic catalyst and a difunctional asymmetric organic catalyst.

5. A process according to claim 4 wherein said catalyst is an organometallic complex of Cu(II) with a chiral 1,2-diamino cyclohexane derivative ligand.

6. A process according to claim 1 wherein step a is carried out in an organic solvent selected among acetonitrile, diethoxyethane, 2,2-dimethoxypropane, 1,2-dimethoxyethane, dioxane, THF and toluene.

7. A process according to claim 1 wherein said reacting is carried out at a temperature comprised between -10°C and 20°C.

8. A process according to one of the preceding claims wherein R is a methyl or ethyl group.

9. A process for preparing a compound of formula 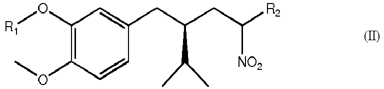 wherein R₁ is a (C₁-C₄)-alkoxy-(C₁-C₄)-alkyl group and R₂ is hydrogen, carboxy, cyano and alkoxy-carbonyl; which comprises
Step (i): oxidizing a primary alcohol of formula 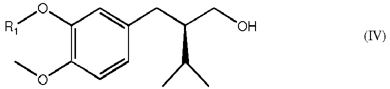 wherein R₁ is defined above, to give an aldehyde of formula 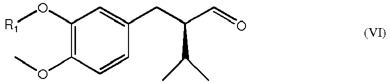 wherein R₁ is defined above;
Step (ii): nitro aldol reacting an aldehyde substrate VI and a nitro derivative of formula 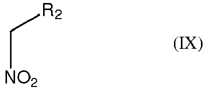 wherein R₂ is defined above; in the presence of a basic species to give a nitro aldol of formula 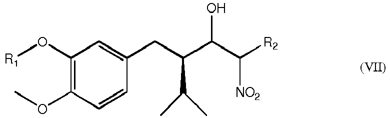 wherein R₁ and R₂ are defined above;
Step (iii): elimination to give a nitroalkene of formula 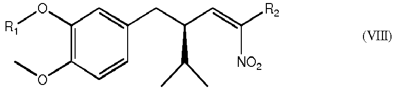 wherein R₁ and R₂ are defined above; and
Step (iv): reducing the conjugated double bond to give a nitroalkane of formula II.

10. A process according to one of the preceding claims wherein R₁ is a methoxypropyl group.

11. A process according to one of the preceding claims wherein R₂ is hydrogen.

12. A process for preparing Boc-lactone which comprises the synthesis of a compound of formula Ia or Ib according to claim 1.

13. A process for preparing aliskiren which comprises the synthesis of a compound of formula Ia or Ib according to claim 1.

14. A compound of formula 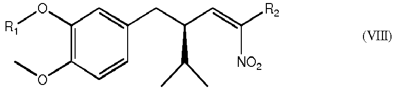 wherein R₁ is a (C₁-C₄)-alkoxy-(C₁-C₄)-alkyl group and R₂ is hydrogen, carboxy, cyano and alkoxy-carbonyl.

15. A compound of formula 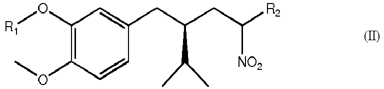 wherein R₁ is a (C₁-C₄)-alkoxy-(C₁-C₄)-alkyl group and R₂ is carboxy, cyano and alkoxy-carbonyl.

## Patentansprüche

1. Verfahren zum Herstellen einer Verbindung mit der Formel 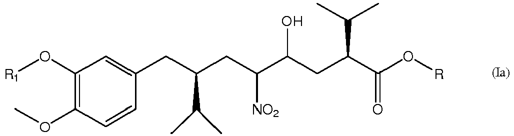 wobei R Wasserstoff, Benzyl und eine gegebenenfalls verzweigte (C₁-C₆)-Alkylgruppe ist und R₁ eine (C₁-C₄)-Alkoxy-(C₁-C₄)-alkylgruppe ist; oder eines Lactonderivats davon mit der Formel 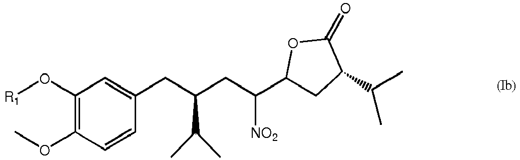 wobei R₁ wie oben definiert ist, welches umfasst:
a. Umsetzen einer Verbindung mit der Formel 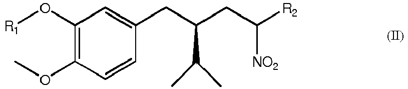 wobei R₁ wie oben definiert ist und R₂ Wasserstoff, Carboxy, Cyano und Alkoxycarbonyl ist; mit einer Verbindung mit der Formel 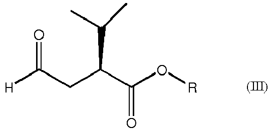 wobei R wie oben definiert ist; in Gegenwart einer basischen Spezies oder eines Katalysators;
b. gegebenenfalls Lactonisieren einer Verbindung der Formel Ia zu einer Verbindung der Formel Ib.

2. Verfahren nach Anspruch 1 wobei die basische Spezies ausgewählt ist aus alkalischen Hydroxiden, alkalischen Alkoxiden, Carbonaten und Salzquellen von Fluoridionen.

3. Verfahren nach Anspruch 2 wobei die basische Spezies Tetrabutylammoniumfluorid ist.

4. Verfahren nach Anspruch 1 wobei der Katalysator ausgewählt ist aus einem organometallischen Katalysator und einem difunktionalen asymmetrischen organischen Katalysator.

5. Verfahren nach Anspruch 4 wobei der Katalysator ein Organometallkomplex von Cu(II) mit einem chiralen 1,2-Diaminocyclohexanderivatliganden ist.

6. Verfahren nach Anspruch 1 wobei Schritt a in einem organischen Lösungsmittel ausgewählt aus Acetonitril, Diethoxyethan, 2, 2-Dimethoxypropan, 1,2-Dimethoxyethan, Dioxan, THF und Toluol durchgeführt wird.

7. Verfahren nach Anspruch 1 wobei das Umsetzen bei einer Temperatur zwischen -10°C und 20°C durchgeführt wird.

8. Verfahren nach einem der vorhergehenden Ansprüche wobei R eine Methyl- oder Ethylgruppe ist.

9. Verfahren zum Herstellen einer Verbindung mit der Formel 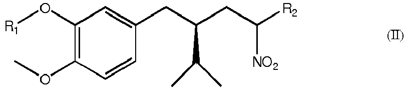 wobei R₁ eine (C₁-C₄)-Alkoxy-(C₁-C₄)-alkylgruppe ist und R₂ Wasserstoff, Carboxy, Cyano und Alkoxycarbonyl ist; welches umfasst Schritt (i): Oxidieren eines primären Alkohols mit der Formel 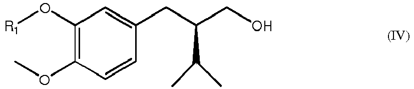 wobei R₁ wie oben definiert ist, um einen Aldehyd mit der folgenden Formel zu ergeben 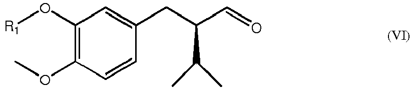 wobei R₁ wie oben definiert ist;
Schritt (ii): Nitroaldolumsetzung eines Aldehydsubstrats VI und eines Nitroderivats mit der Formel 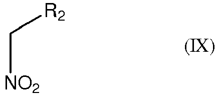 wobei R₂ wie oben definiert ist; in Gegenwart einer basischen Spezies, um ein Nitroaldol mit der folgenden Formel zu ergeben 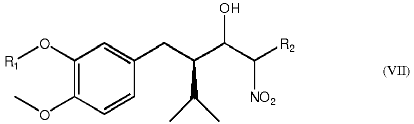 wobei R₁ und R₂ wie oben definiert sind;
Schritt (iii): Eliminierung, um ein Nitroalken mit der folgenden Formel zu ergeben 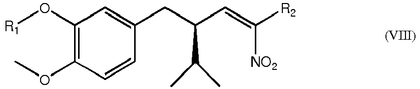 wobei R₁ und R₂ wie oben definiert sind; und
Schritt (iv): Reduzieren der konjugierten Doppelbindung, um ein Nitroalkan der Formel II zu ergeben.

10. Verfahren nach einem der vorhergehenden Ansprüche wobei R₁ eine Methoxypropylgruppe ist.

11. Verfahren nach einem der vorhergehenden Ansprüche wobei R₂ Wasserstoff ist.

12. Verfahren zur Herstellung von Boc-Lacton das die Synthese einer Verbindung mit der Formel Ia oder Ib gemäß Anspruch 1 umfasst.

13. Verfahren zur Herstellung von Aliskiren das die Synthese einer Verbindung mit der Formel Ia oder Ib gemäß Anspruch 1 umfasst.

14. Verbindung mit der Formel 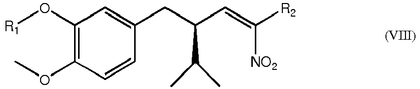 wobei R₁ eine (C₁-C₄)-Alkoxy-(C₁-C₄)-alkylgruppe ist und R₂ Wasserstoff, Carboxy, Cyano und Alkoxycarbonyl ist.

15. Verbindung mit der Formel 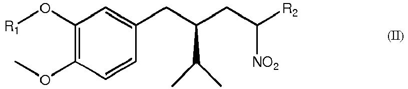 wobei R₁ eine (C₁-C₄)-Alkoxy-(C₁-C₄)-alkylgruppe ist und R₂ Carboxy, Cyano und Alkoxycarbonyl ist.

## Revendications

1. Procédé pour préparer un composé de formule 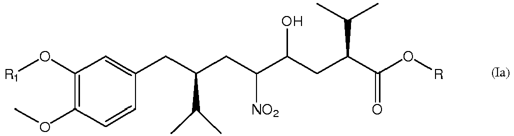 dans laquelle R représente hydrogène, un groupe benzyle et un groupe (C₁-C₆)-alkyle éventuellement ramifié et R₁ représente un groupe (C₁-C₄)-alcoxy-(C₁-C₄)-alkyle ; ou un dérivé de type lactone de celui-ci de formule 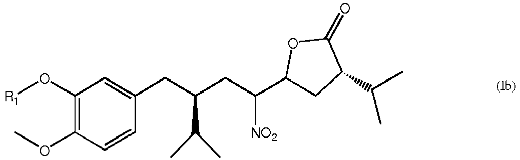 dans laquelle R₁ est défini ci-dessus, qui comprend
a. la réaction d'un composé de formule 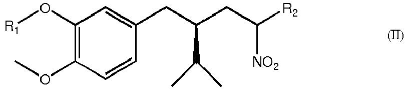 dans laquelle R₁ est défini ci-dessus et R₂ représente hydrogène, carboxy, cyano et alcoxycarbonyle ; avec un composé de formule 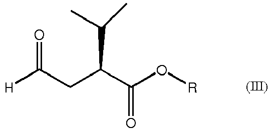 dans laquelle R est défini ci-dessus ; en présence d'une espèce basique ou d'un catalyseur ;
b. lactonisation éventuelle d'un composé de formule Ia en un composé de formule Ib.

2. Procédé selon la revendication 1 ladite espèce basique étant choisie parmi les hydroxydes alcalins, les alcoxydes alcalins, les carbonates et les sels sources d'ions fluorure.

3. Procédé selon la revendication 2 ladite espèce basique étant le fluorure de tétrabutylammonium.

4. Procédé selon la revendication 1 ledit catalyseur étant choisi parmi un catalyseur organométallique et un catalyseur organique asymétrique difonctionnel.

5. Procédé selon la revendication 4 ledit catalyseur étant un complexe organométallique du Cu (II) avec un ligand chiral dérivé du 1,2-diaminocyclohexane.

6. Procédé selon la revendication 1 l'étape a étant effectuée dans un solvant organique choisi parmi l'acétonitrile, le diéthoxyéthane, le 2,2-diméthoxypropane, le 1,2-diméthoxyéthane, le dioxane, le THF et le toluène.

7. Procédé selon la revendication 1 ladite réaction étant effectuée à une température comprise entre -10°C et 20°C.

8. Procédé selon l'une quelconque des revendications précédentes R représentant un groupe méthyle ou éthyle.

9. Procédé pour préparer un composé de formule 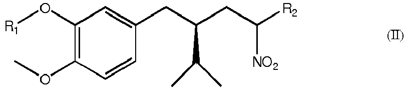 dans laquelle R₁ représente un groupe (C₁-C₄)-alcoxy-(C₁-C₄)-alkyle et R₂ représente hydrogène, carboxy, cyano et alcoxycarbonyle ; qui comprend Étape (i): oxydation d'un alcool primaire de formule 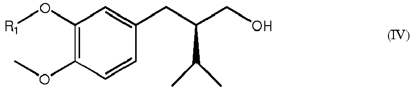 dans laquelle R₁ est défini ci-dessus, pour obtenir un aldéhyde de formule 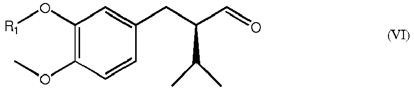 dans laquelle R₁ est défini ci-dessus ;
Étape (ii): réaction nitroaldol d'un substrat d'aldéhyde VI et d'un dérivé nitro de formule 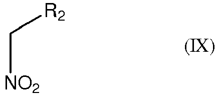 dans laquelle R₂ est défini ci-dessus ; en présence d'une espèce basique de manière à obtenir un nitroaldol de formule 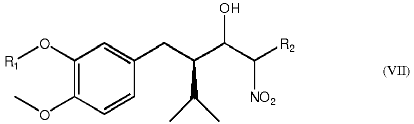 dans laquelle R₁ et R₂ sont définis ci-dessus ;
Étape (iii): élimination pour obtenir un nitroalcène de formule 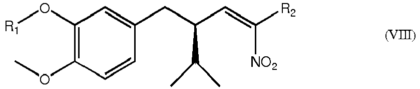 dans laquelle R₁ et R₂ sont définis ci-dessus ; et
Étape (iv): réduction de la double liaison conjuguée pour obtenir un nitroalcane de formule II.

10. Procédé selon l'une quelconque des revendications précédentes R₁ représentant un groupe méthoxypropyle.

11. Procédé selon l'une quelconque des revendications précédentes R₂ représentant hydrogène.

12. Procédé pour préparer une Boc-lactone qui comprend la synthèse d'un composé de formule Ia ou Ib selon la revendication 1.

13. Procédé pour préparer de l'aliskirène qui comprend la synthèse d'un composé de formule Ia ou Ib selon la revendication 1.

14. Composé de formule 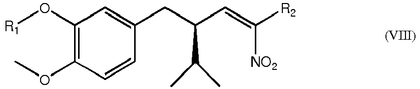 dans laquelle R₁ représente un groupe (C₁-C₄)-alcoxy-(C₁-C₄)-alkyle et R₂ représente hydrogène, carboxy, cyano et alcoxycarbonyle.

15. Composé de formule 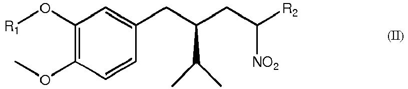 dans laquelle R₁ représente un groupe (C₁-C₄)-alcoxy-(C₁-C₄)-alkyle et R₂ représente carboxy, cyano et alcoxycarbonyle.
